# EUROPEAN PATENT APPLICATION

(11) **EP 3 967 738 A1**
(43) Date of publication of application: **16.03.2022**
(21) Application number: 21194718.9
(22) Date of filing: 03.09.2021
(51) Int. Cl.: C10G 7/12, C10G 47/36, G01N 33/28, G05B 13/04, G06N 3/08, G16C 20/30

(54) **ESTIMATION METHOD OF NITROGEN CONTENT IN THE HYDROCRACKER REACTOR FEEDSTOCK FOR TEMPERATURE OPTIMIZATION**

(30) Priority: 15.09.2020 TR 202014625
(71) Applicant: SOCAR TURKEY ENERJI A.S., SARIYER/ ISTANBUL (TR)
(72) Inventor: BAYAR, Ibrahim, 34485 Istanbul (TR); NASIRLI, Emin, 34485 Istanbul (TR); KAPLAN, Kemal Burcak, 34485 Istanbul (TR); KARAN, Osman Kubilay, 34485 Istanbul (TR); SARIALP, Gokhan, 34485 Istanbul (TR); ALIYEV, Mammad, 34485 Istanbul (TR); DOGRU, Atilla, 34485 Istanbul (TR); GOREN, Mustafa, 34485 Istanbul (TR); AYHAN, Umut Baris, 34485 Istanbul (TR); YILDIZ, Onur, 34485 Istanbul (TR); ERISEN, Ender, 34485 Istanbul (TR)
(74) Representative: Sevinç, Cenk

(57) **Abstract**

The present invention relates to an estimation system and method of nitrogen that enables estimation of the potential nitrogen amount present in reactor feed minimum 5 hours beforehand by analyzing the process variables and refinery raw material properties. The invention is particularly related to a nitrogen estimation method that provides the required pre-optimization in the reactor according to the pre-estimated nitrogen values and suggests optimum temperature values for reactor optimization by analyzing the correlation between nitrogen and the reactor variables.

## Description

### Technical Field of the Invention:

The present invention relates to an estimation system and method of nitrogen that enables estimation of the potential nitrogen amount present in reactor feed minimum 5 hours beforehand by analyzing the process variables and refinery raw material properties.

The present invention is particularly relates to a nitrogen estimation method that provides the required pre-optimization in the reactor according to the pre-estimated nitrogen values and suggests optimum temperature values for reactor optimization by analyzing the correlation between nitrogen and the reactor variables.

### State of the Art:

The aim of the refining process is to process natural raw materials and convert them into usable products. The products obtained from refineries can be classified as fuel for cars, trucks, airplanes, vessels and other vehicles, fuel for producing heat and energy to be used commercially or domestically, raw materials for petrochemistry and the chemistry industry, special products such as lubricant oils, paraffin/wax and bitumen and byproducts such as energy, heat (vapor) and power (electricity).

The combination of processing units that convert raw materials into products by auxiliary plants and facilities is called refining. The basic production units of the refineries are processing units and auxiliary plants. In addition to these units, facilities that produce lubricant oils, base oil complexes, petrochemical or aromatic raw materials are also present in refineries.

The main sections of the refinery complex can generally be listed as the crude oil and vacuum distillation unit, LPG amine unit, naphtha hydrodesulfurizer, isomerization and reformer unit, kerosene and diesel hydrodesulfurizer unit, kerosenemerox unit, propane-butane separation unit, sulfur recovery unit, catalytic cracking unit and the hydrocracker unit.

Hydrocracking (hydrocracker) reactions, in which heavy petroleum fractions are converted into valuable products such as kerosene, diesel, gasoline, LPG, under high temperature and pressure, in a dense hydrogen environment, is one of the most important processes of refineries.

The hydrocracker unit consists of two main parts, the reactor part, where the reactions occur, and the separation part, where the products formed are separated from each other. The load is subjected to pre-processing in order to remove sulfur, nitrogen and heavy metals. Following this the hydrocracking reactions start, where the products are formed. Hydrocracking is carried out by cracking and hydrogen saturation. While cracking reactions require a small amount of heat, a high amount of heat is released during hydrogen saturation. Therefore, temperatures begin to increase together with hydrocracking reactions. These temperature increases are controlled by the cold hydrogen quenches given between the reactor beds.

The purpose of the hydrocracker unit is to increase the yield of white products by converting heavy vacuum oil into lighter products (low molecular weight) (fuel gas, light-heavy naphtha, kerosene, LPG and diesel oil) in a hydrogen environment with the help of high temperature and pressure. Generally there are two types of reactions in hydrocracker units. These are the cleaning (purification) reaction and the hydrogen cracking (hydrocracking) reaction. During the cleaning reactions, the impurities in the load are separated, and during the hydrogen cracking reactions, more valuable and lighter products are obtained. The heavy vacuum oil and hydrogen are heated in heat exchangers and are delivered to the charging furnace, where they are heated to the required temperature in order to carry out reactions. Here, olefin saturation, aromatic saturation, desulfurization, deoxygenation and nitrogen removal reactions occur.

The unexpected nitrogen increases in the hydrocracker reactor feed, can result in temporary nitrogen poisoning. Following nitrogen poisoning, the reactor's cracking ability is reduced, thereby resulting in reduction of the refinery capacity.

For this reason, it is of great importance to determine and track the amount of nitrogen in the hydrocracker feed. A number of applications are used in order to be able to monitor the nitrogen in the hydrocracker feed.

Some of the applications that are used to monitor nitrogen are measuring the nitrogen value in real time using a nitrogen analyzer in flows received by the hydrocracker, measuring the nitrogen value several times a day using laboratory analyzes, and calculating the real-time nitrogen value using the reactor kinetic model/digital twin.

The optimization of the refinery is also crucial in order to be able to monitor the nitrogen available in the hydrocracker feed. Although refinery optimization methods are available in the state of the art, these methods are not related to hydrocrackers. Additionally there are some nitrogen measuring devices available, but these devices are used in different fields and they only allow measurement.

In the state of the art, the patent document numbered **"2010/06134"** and titled "Landmine detection method and device using nitrogen gas measurement" was examined. In the abstract section of the invention subject to the application, it is stated that "The invention is related to a device and method for landmine detection using nitrogen gas measurements. The device consists of a sensor tip, an air analysis sensor, a conductive cable, a processor unit, a processor instrument panel, a processor keypad, a warning lamp and processor unit, pocket clamp parts. The method consists of the process steps of measuring the amount of inorganic nitrogen in the environment by means of the sensors available on the device, sending the obtained data to the processor unit via the conductive cable, detecting the mine in case the measurement value exceeds the predefined critical level."

In the state of the art, the patent document numbered **"2014/14554"** titled "A decision support system that enables product transfer optimization among refineries" was examined. In the abstract section of the invention subject to the application, it is stated that "A decision support system is developed, which enables optimization of the product transfers between refineries. Said decision support system includes at least one data entry unit that enables users to enter transport parameters; at least one optimization solver unit, which determines the transportation route with minimum transportation cost by calculating the costs for different transportation routes in line with the transportation parameters entered from the data entry unit; at least one data output unit that presents, the product based transport route determined by the optimization solver unit to the user; at least one output control unit in which the suitability of the proposed transport route from the data output unit is checked by the user and the transport parameters are re-entered if the transport route is not suitable, and the suitability information is entered by the user if the transport route is suitable; and if the presented transport route is suitable it includes at least one data recording unit wherein the transport route is recorded as the final result, and in addition to these, at least one database unit that is associated with said data entry unit where the fixed parameters are registered".

The patent document numbered **"**EP1794584A1**"** in the state of the art was examined. In the invention that is the subject of the application, a portable apparatus used for the analysis of a refined raw material or a product belonging to a refinery process is mentioned. This analysis device can provide combined micro ion mobility / differential mobility spectrometry, nitrogen and sulfur analysis.

The patent document numbered **"**CN106568816A**"** in the state of the art was examined. In the invention subject to the application, the reactive method used to be able to rapidly measure the ammonia nitrogen content of the oil treatment sewer system using an electrode method is described. This method has been noted to be very suitable for measuring the ammonia nitrogen content of the reactive, oil refining sewer system

In the state of the art, the late arrival of nitrogen values in the laboratory method used for nitrogen measurement does not provide sufficient time for reactor optimization. The personnel and/or the measuring device performing analysis have a margin of error. The manpower/time and costs spent for analysis increases. Moreover, the nitrogen values are measured with laboratory analysis 2-3 times, and this leads to loss of time.

In the state of the art, sufficient time cannot be provided for real-time measurement reactor optimization in analyzers used for nitrogen measurement. The price of the analyzer devices is quite high. Also the manpower/time and costs spend for periodical calibration and maintenance of analyzers also increase. The error rate of the analyzer increases in time due to insufficient calibration and deterioration of the parts of the analyzer in time.

In the state of the art, sufficient time cannot be provided for real-time measurement reactor optimization in the reactor kinetic model/digital twin used for nitrogen measurement. Such models have complex development and calibration processes.

Consequently, the disadvantages disclosed above and the inadequacy of available solutions in this regard necessitated making an improvement in the relevant technical field.

### Objects of the Invention

The most important object of the present invention is to ensure that the estimation model of the method can estimate the potential nitrogen amount present in reactor feed by analyzing the process variables and refinery raw material properties. It is ensured that the optimizations of the reactor are carried out beforehand according to the nitrogen value that is estimated prior to formation.

Another object of the present invention is to prevent catalyzer poisoning as a result of the optimization carried out and as a result, to prevent refinery production loss.

Yet another object of the present invention is to ensure that the computer can suggest optimum temperature values for reactor optimization by analyzing the correlation between the reactor variables and the nitrogen value.

Yet another object of the present invention is to provide an estimation of the nitrogen value in the reactor feed at least 5 hours in advance.

Yet another object of the present invention is to provide sufficient time for reactor optimization by means of pre-estimating the nitrogen in the feed.

Yet another object of the present invention is to ensure that the personnel at the factory who uses the method outputs can optimize the reactor in order to operate with new nitrogen values.

Yet another object of the present invention is to ensure that the malfunction of the model is prevented by means of re-training and calibration/tuning based on previous data.

Yet another object of the present invention is to develop an estimation value over historical data by means of using machine learning algorithms.

Yet another object of the present invention is to ensure that the model performance tracking system continuously measures model performance and that it can calibrate/tune the model at certain intervals.

Yet another object of the present invention is to enable the relevant personnel in the factory using the outputs of the method to optimize the reactor to operate at the nitrogen value that will be obtained after 5 hours.

The structural characteristic features and all of the advantages of the invention shall be understood more clearly by means of the detailed description given below. Therefore if an evaluation is to be carried out, it should be done by taking the figures and this detailed description into consideration.

### Description of the Invention

The estimation model used by the system and method of the invention can pre-estimate the potential nitrogen amount present in the hydrocracker reactor feed by analyzing the process variables and refinery raw material properties and it enables the required pre-optimization to be carried out according to the pre-estimated nitrogen value. By this means, the nitrogen amount in the raw material for temperature optimization of the hydrocracker reactor can be pre-estimated.

The raw material properties of the refinery and the process variables are values of density of the crude petrol, which is a refinery raw material, the boiling point, nitrogen combination values thereof and data received from the level sensors of tanks, columns and other equipment used in the process and the temperature, pressure and flow values of the delayed cocker unit and crude distillation unit prior to hydrocracking. The flows of these units enter the hydrocracker.

The estimation system subject to the invention comprises sensors that enable to collect data, a database in which the information collected from the sensors are stored, and a computer including a processor that enables to carry out estimation based on the information received from the sensors. The computer carried out an estimation process according to the data it receives using machine learning models and artificial intelligence. Besides the data received from the temperature, pressure, flow and level sensors, the laboratory analysis results received from the raw material tanks are used for estimation.

The estimation method performed by the computer is enabled with certain process steps. These process steps in general are, collecting data, examining data, determining optimum model by machine learning, and application of the determined data to the factory. The estimation method is basically formed of two primary process steps. The first process step is the creation of a model by the computer and the second process step is to be able to perform estimation according to the created model.

The below mentioned process steps need to be carried out in order to create a model by the method that operates on a computer and that enables pre-estimation of the potential nitrogen amount in the hydrocracker reactor feed.

First of all the first model is formed. This procedure is carried out for each factory/process once. After the model is formed, it is put to use, and new modeling is not required. The density, boiling point and nitrogen component values of crude petrol which is a raw material of the refinery are measured and these measurements are recorded to the database. The level sensor data of raw material tanks are recorded to the database. Sensor data such as the temperature, pressure flow, and level of the delayed cocker unit and the crude distillation unit prior to the hydrocracker, are recorded to the database. All recorded data are obtained by the computer from the database and a dataset is created. The erroneous values obtained from the sensors are deleted from the computer and the data set at this stage. The variables that are important for the model are determined by the computer. At this stage selected data science methods are used.

As a result of the examination of the time-dependent correlations between the variables that are important for the model and the analysis of the dynamics in these variables, time shifting is performed in the data set. 80% of the dataset was used for training and for validation of the model, and the remaining 20% was used for testing. The most suitable neural network structure was selected according to the R2 and rmse (root mean square error) performance values of the tested models. Algorithms developed based on the shape value method were used for the analysis of the neural network model results. A system that provides a temperature suggestion for reactor optimization has been established by using these analysis results.

A neural network self-tuning mechanism that is to be used by the computer has been developed. By means of this mechanism the model can self-tune the results at certain intervals in compliance with the new data available in the database. A user interface is used to reflect the model estimation results and optimization suggestions. Finally the model to use so that it can operate on the computer or on a cloud server.

After the model of the method, which operates on the computer and allows estimating the potential amount of nitrogen in the hydrocracker reactor feed, is established, the following steps are carried out by the computer in order to perform estimation.

First of all, the variables, which are model inputs, are automatically uploaded to the computer from the database. The correction (error, null value etc.) test of the data obtained is carried out by the computer. The computer operates the estimation algorithm and sends the estimation results to the user interface. The information sent to the interface is the nitrogen value information that shall be formed 5 hours later. The suitable reactor temperatures according to nitrogen estimation are calculated by the computer and are sent to the user interface. The computer compares the model estimations with real values. If the difference between the values increases in time, the computer enables automatic calibration of the model. The model that is calibrated is put into use following the confirmation by the user. Finally the computer reports the daily and weekly model estimation and suggestion outputs and model performance (R2 and rmse) and automatically shares this information with the related people.

In the method of the invention, the amount of nitrogen in the reactor feed is measured by sensors and analysis. The measured nitrogen amounts are transmitted to the database that is connected to the computer. The database can be the computer's own database, or it can be a database on a remote server. The nitrogen value amount data obtained from the database is analyzed by the computer. All of the process variables shall be analyzed during data analysis and the most suitable ones for the model are selected. Among such analysis methods such as correlation, principal component analysis (PCA), model based feature selection (linear and/or nonlinear ensemble models) and recursive feature elimination are used. As the process is dynamic, the time dependent correlations between the process variables during data analysis has been examined and the dynamics within the variables that are important for the model has been analyzed and time shifting in data has been carried out. Data analysis and procedures such as the trial of algorithms are carried out during the modeling stage. The modeling procedure is performed again for all new factory/processes to which this algorithm shall be applied. After the algorithm, which is suitable as a result of the modeling, is saved and put into use, estimation results and optimization suggestions can be given using up-to-date process data. The estimation model, which has been modeled and put into use, is calibrated periodically according to current data, as there is no need for continuous remodeling.

The computer tries different algorithms and models. An optimum algorithm is selected according to the tried machine learning models. As the computer continuously calculates the correlation between the model reactor temperature and the nitrogen value in the feed, for example when the nitrogen value that is a result of estimation increases by 10 units, a suggestion can be given on how much the reactor temperatures should be increased. The models applied here are machine learning models. Neural network model has been used for modeling. The data set was divided as 80/20%. 80% of the dataset was used for training and for validation of the model, and the remaining 20% was used for testing. The most suitable neural network was selected according to the R2 and rmse (root mean square error) performance values of the tested models. The selected optimum model is applied to the refinery. Thereby, the computer estimates the nitrogen value in the reactor feed at least 5 hours in advance. Since the estimation is 5 hours before the formation, sufficient time is provided for reactor optimization. Additionally, the computer analyzes the correlation between the reactor variables and the nitrogen value and optimum temperature values for reactor optimization are suggested. During the modeling phase, the neural network algorithm learns the connections between the process variables. Since the NN model is a black box, the model cannot reflect the connection between variables with a formula such as a linear regression algorithm. Therefore, in the method of the invention, the results of the neural network-based black box model are analyzed using shape value-based algorithms.

An estimation value over historical data is developed as the method subject to the invention uses machine learning. The refinery raw material properties and the process variables in the refinery units prior to hydrocracking are used as model variables. The model performance tracking system that has been developed and used by the computer, continuously measures the model performance and tunes the model periodically. Catalyzer poisoning is prevented as a result of the optimization carried out by the method subject to the invention and as a result the refinery production loss is minimized.

## Claims

1. An estimation method that operates on a computer that enables to estimate the potential nitrogen amount in the hydrocracker reactor feed beforehand **characterized in that**, it comprises the process steps of;
- Creating the first model,
- Measuring the density, boiling point and nitrogen component values of crude petrol which is a raw material of the refinery and recording these measurements to the database,
- Recording the level sensor data of raw material tanks to the database,
- Recording sensor data such as the temperature, pressure flow, and level of the delayed cocker unit and the crude distillation unit prior to the hydrocracker, to the database,
- Obtaining all recorded data by the computer from the database and creating a dataset,
- Determining the variables that are important for the model by the computer,
- Putting the model to use so that it can operate on the computer, or on a server,
- Automatically uploading the variables that are model inputs to the computer from the database,
- Carrying out correction testing of the obtained data by the computer,
- Operating the computer estimation algorithm and sending the estimation results to the user interface,
- Calculating the suitable reactor temperatures according to nitrogen estimation and sending this information to the user interface,
- Comparing the model estimations with real values by the computer,
- Sharing automatically to the related people and reporting by the computer, the daily and weekly model estimation and suggestion outputs and model performance.

2. An estimation method according to Claim 1, **characterized in that**, the process step of obtaining all recorded data by the computer from the database and creating a dataset comprises the process step of deleting the erroneous values received from the sensors form the data set by the computer.

3. An estimation method according to Claim 1, **characterized in that**, in the process step of comparing by computer the model estimations with real values, automatic calibration is carried out by the computer if the difference between the values increases in time.

4. An estimation method according to Claim 1, **characterized in that** the process step of determining the variables that is important for the model by the computer, comprises the process steps of;
∘ Examining the time-dependent correlations between the variables that are important for the model,
∘ Performing time shifting in the data set as a result of the analysis of the dynamics in these variables,
∘ Using 80% of the dataset for training and for validation of the model, and using the remaining 20% for testing,
∘ Selecting the most suitable neural network structure according to the R2 and rmse (root mean square error) performance values of the tested models,
∘ Establishing a system that provides temperature suggestion for reactor optimization from the results of the neural network-based black box model using shape value-based algorithms,
∘ Self-tuning of the model according to the new results in the database periodically as a result of the neural network self-tuning mechanism that is to be used by the computer.

5. An estimation method according to Claim 1, **characterized in that**, in the process step of operating by the computer the estimation algorithm and sending the estimation results to the user interface; the computer selects the optimum algorithm according to the tried machine learning models.

6. An estimation method according to Claim 1 or Claim 5, **characterized in that** in the process step of operating by the computer the estimation algorithm and sending the estimation results to the user interface; the computer is able to suggest how much the reactor temperatures should be increased according to the nitrogen value obtained following estimation by continuously calculating the correlation between the model reactor temperature and the nitrogen value in the feed.

7. An estimation method according to Claim 1, **characterized in that** in the process step of operating by the computer the estimation algorithm and sending the estimation results to the user interface; the computer uses the neural network model for modeling.
